# EUROPEAN PATENT APPLICATION

(11) **EP 4 850 805 A1**
(43) Date of publication of application: **09.12.2026**
(21) Application number: 25747725.7
(22) Date of filing: 23.01.2025
(51) Int. Cl.: B01J 13/16

(54) **BIOMICROSPHERE SUITABLE FOR LARGE-SCALE PRODUCTION AND PREPARATION METHOD THEREFOR**

(30) Priority: 31.01.2024 CN 202410140286
(71) Applicant: Kangma-Healthcode (Shanghai) Biotech Co., Ltd, Pudong New Area Shanghai 201321 (CN)
(72) Inventor: GUO, Min, Shanghai 201321 (CN); YAN, Zhe, Shanghai 201321 (CN); LI, Ning, Shanghai 201321 (CN); YU, Xue, Shanghai 201321 (CN)
(74) Representative: Cameron Intellectual Property Ltd
(86) International application number: PCT/CN2025/074118
(87) International publication number: WO 2025/162155

(57) **Abstract**

The present invention provides a biomicrosphere suitable for large-scale production and a preparation method therefor. A polymer with a known molecular weight is directly bound onto the surface of a biomicrosphere body, so that large-scale production of biomicrospheres becomes feasible. In addition, the length of the polymer on the surface of the microsphere can be accurately controlled, so that biomicrospheres having uniform particle sizes and stable adsorption capacity can be obtained.

## Description

### TECHNICAL FIELD

The present disclosure belongs to the technical field of biochemistry, and specifically relates to a method for large-scale preparation of a biological purification microsphere.

### BACKGROUND

Currently, in the field of separating biomolecular targets, among stationary phases of traditional chromatography columns, organic stationary phase (agarose, etc.) packing materials have problems such as poor pressure resistance; inorganic stationary phases have relatively high pressure resistance, but have a relatively small loading capacity.

In addition, currently, microsphere products used for biomolecular separation have relatively small particle sizes, are very easily clogged in a packed column, resulting in reduced separation efficiency, and are not suitable for the separation of relatively large biomolecules.

Therefore, both inorganic materials and organic stationary phases need further performance improvement.

In addition, traditional inorganic materials have excellent mechanical performances and chemical corrosion resistance, and are widely used in various fields of modern industry and agriculture. By performing polymer grafting modification on the surface of an inorganic material, and by regulating the monomer component, molecular weight, topological structure, etc. of the grafted polymer chain, various materials science properties of traditional inorganic materials can be significantly improved; particularly in the field of biomedicine, the repeatability and reliability of large-scale production processes can be significantly improved by using such materials as separation and purification media, and separating and purifying proteins or protein drugs through different separation and purification methods. In addition to the properties of the inorganic material itself, the grafting-modified polymer chain plays a decisive role in the final separation and purification performance; therefore, the industry has been exploring technologies capable of quantitatively grafting polymer chains for large-scale production.

From the perspective of existing synthesis techniques, in the field of biomolecular separation, the synthesis process of polymer-grafted inorganic materials generally involves functionalizing the surface of the inorganic material with a silane coupling agent, and then further modifying the functional groups, constructing active sites on the surface of the inorganic material to initiate a polymerization reaction, thereby achieving the purpose of grafting polymer chains onto the surface of the inorganic material; the second synthesis process relies on specific chemical reactions, and through chemical reactions between specific chemical functional groups (on the polymer backbone or side chain) and chemical functional groups on the surface of the inorganic material, polymer chains with clearly defined chemical structures, polymer molecular weights, and topological structures are quantitatively grafted onto the surface of the inorganic material. Because various polymers have been grafted onto the surface of inorganic materials through controlled radical polymerization techniques, polymerization techniques such as atom transfer radical polymerization (ATRP), reversible addition-fragmentation chain transfer polymerization (RAFT), and nitroxide-mediated radical polymerization (NMP) have been widely adopted in the field of academic research, and various polymers with clearly defined chemical structures, polymer molecular weights, and topological structures have been constructed on the surfaces of various inorganic materials. However, such methods are limited by the relatively low radical concentration during the reaction process and the additional increased costs (catalyst, chain transfer agent, or radical stabilizer), and achievements in their industrial scale-up production have been rarely reported in academia and industry. Moreover, such methods have difficulty in controlling the chain length of the polymer on the microsphere surface, thereby resulting in non-uniform microsphere surfaces, unstable particle sizes, and relatively large loading capacity differences between different batches of microspheres. The present disclosure provides a solution precisely for solving the above technical problems.

### SUMMARY

The purpose of the present disclosure is to overcome the aforementioned problems existing in the prior art, and to provide a biomicrosphere capable of large-scale production and preparation, as well as a large-scale production method and application thereof.

Through the method of the present application, large-scale production of a biomicrosphere may be realized, and the length of the linear polymer on the microsphere surface may be accurately controlled, thereby obtaining a biomicrosphere with uniform particle size and stable adsorption capacity.

A first aspect of the present disclosure provides a biomicrosphere, wherein an outer surface of a microsphere body of the biomicrosphere is covalently coupled with at least one polymer chain, and other portions of the polymer chain are unattached to the outer surface of the microsphere body; the polymer chain contains a functional group, and the functional group specifically binds to a biological target via a specific binding site, wherein the polymer chain comprises at least one segment, each of the at least one segment is derived from a polymer, and the at least one segment of the polymer chain is derived from a polymer having a molecular weight > 5000; the particle size of the biomicrosphere body is ≥ 10 µm.

In another preferred embodiment, the polymer is a linear polymer, preferably, the linear polymer has a branched chain, and the end of the branched chain contains the functional group; and/or
the functional group of the polymer is one of carboxyl, hydroxyl, amino, and sulfhydryl or a combination thereof.

In another preferred embodiment, a monomer of the polymer is an acrylic polymer, an acrylic copolymer, an amino acid polymer, or other carboxylic acid polymers; preferably, a monomer unit of the acrylic polymer comprises one of acrylic acid, acrylate salt, acrylate ester, methacrylic acid, methacrylate salt, and methacrylate ester or a derivative thereof, or any combination of the above.

In another preferred embodiment, the functional group is linked to the specific binding site;
preferably, the specific binding site comprises a nickel ion, biotin, a biotin analog, avidin, an avidin analog, an antibody-type tag, or an antigenic tag.

In another preferred embodiment, the molecular weight of the polymer is: greater than or equal to 50,000, greater than or equal to 150,000, 450,000-6,000,000, or 450,000-4,500,000, preferably, the molecular weight of the linear polymer is 50,000, 150,000, 200,000, 300,000, 400,000, 500,000, 600,000, 700,000, 800,000, 900,000, 1,000,000, 1,250,000, 1,500,000, 1,800,000, 2,000,000, 2,500,000, 3,000,000, 3,500,000, 4,000,000, 4,500,000, 5,000,000, or 6,000,000; and/or
the particle size of the biomicrosphere body is 10 µm-600 µm, 30 µm-600 µm, greater than or equal to 30 µm and less than 600 µm, preferably, the particle size of the biomicrosphere body is 10 µm, 40 µm, 50 µm, 60 µm, 70 µm, 80 µm, 90 µm, 100 µm, 120 µm, 150 µm, 200 µm, 300 µm, 400 µm, and 600 µm.

In another preferred embodiment, the biomicrosphere is free of network cross-linking.

In another preferred embodiment, the coupling of the polymer chain to the surface of the microsphere body is achieved by covalently coupling the polymer which is known to the outer surface of the microsphere body;
for a polymer chain having a plurality of the segments, one known polymer is covalently coupled to the outer surface of the microsphere body and then linked to another known polymer.

In another preferred embodiment, the biomicrosphere further has any one or more of the following features:
(1) when the polymer chain comprises a plurality of segments, the polymer from which each segment is derived is the same or different;
(2) the microsphere body is a solid microsphere;
(3) the microsphere body is a glass microsphere or a microsphere containing a magnetic material.

A second aspect of the present disclosure provides a method for preparing a biomicrosphere, wherein a known polymer is covalently coupled to a surface of a microsphere body to achieve covalent coupling between the surface of the microsphere body and a polymer chain;
preferably, no crosslinking agent is used and/or the polymer is a linear polymer.

In another preferred embodiment, the method comprises the following steps:
(1) subjecting the biomicrosphere body to chemical modification, introducing amino or other groups capable of binding to carboxyl onto the outer surface of the biomicrosphere body, to form a biomicrosphere A;
   preferably, the biomicrosphere body is chemically modified using a silane coupling agent;
(2) under the condition of no crosslinking agent, linking a polymer to a surface of the biomicrosphere A to obtain a biomicrosphere B.

In another preferred embodiment, the method for preparing the biomicrosphere comprises the following steps:
linking the silane coupling agent to the polymer, and then linking the resulting product to the biomicrosphere body to obtain the biomicrosphere B.

In another preferred embodiment, the method for preparing the biomicrosphere further comprises:
(3) under the condition of no crosslinking agent, further linking other polymers to the surface of the biomicrosphere B to obtain a biomicrosphere D.

In another preferred embodiment, the method further comprises the following steps: carrying out a reaction of linking a functional group of the polymer to a specific binding site, preferably, using the biomicrosphere B or the biomicrosphere D to carry out the reaction of linking the functional group of the polymer to the specific binding site.

In another preferred embodiment, in the large-scale preparation method for the biomicrosphere, the biomicrosphere B or the biomicrosphere D is coupled with tricarboxyamine, and then complexed with Ni ions to obtain a biomicrosphere C;
or, biotin, a biotin analog, avidin, an avidin analog, an antibody-type tag, or an antigenic tag is linked to the functional group;
preferably, the tricarboxyamine is N,N-bis(carboxymethyl)-L-lysine, nitrilotriacetic acid, and a combination thereof.

In another preferred embodiment, the ratio of the usage amount of the linear polymer to the biomicrosphere body is: 8 × 10⁻⁷ mol/10 mL-2.0 × 10⁻⁵ mol/10 mL.

A third aspect of the present disclosure provides a method for separating a biological target, wherein the biomicrosphere according to the first aspect of the present disclosure is used to bind to the biological target to be separated.

In another preferred embodiment, the biological target is one or more of a protein, a nucleic acid, a cell, a polysaccharide, and a peptide.

A fourth aspect of the present disclosure provides a separation system, wherein the separation system uses the biomicrosphere according to the first aspect, or uses the biomicrosphere prepared by the method according to the second aspect for separation.

Compared with the prior art, the present disclosure has the following beneficial effects or advantages:
(1) The biomicrosphere provided by the present disclosure has a relatively large particle size, which facilitates large-scale preparation and use. Compared with currently conventional nano-sized and several-micron-sized biomicrospheres, the biomicrosphere body provided by the present disclosure has the large-size characteristic of greater than or equal to 10 microns (even capable of reaching several hundred microns), and the size further increases after linking polymer chains, thus providing better visibility during operation and facilitating operation during modification of the microsphere; in the subsequent purification and separation process, because the pores between large particles are relatively large, the feed liquid, impurities, and the like are less likely to clog the chromatography column during column packing, and elution and regeneration are facilitated, which can not only avoid reduction in separation efficiency caused by retention, but also be suitable for the separation of relatively large biomolecules, and suitable for large-scale separation, purification and production; further, the adsorption effect can be more effectively ensured in combination with a polymer chain of a certain molecular weight.
(2) The biomicrosphere provided by the present disclosure has a good function of adsorbing and purifying biological macromolecules such as proteins. Although the large particle size objectively results in a relatively smaller specific surface area of the microspheres, the high degree of polymerization of the linked polymers reduces steric hindrance and increases the binding opportunities with specific sites. Furthermore, for the linked linear polymers, the long backbone allows the functional groups thereon to bind to specific binding sites (also referred to as purification media). Consequently, the specific adsorption capacity for biological macromolecules such as proteins is significantly enhanced, and a high overall adsorption capacity can still be achieved. Therefore, while accommodating large-scale production, the separation efficiency is maintained at a high level, realizing high-throughput separation.
(3) The linkage on the surface of the biomicrosphere provided by the present disclosure employs a polymer with a known degree of polymerization, i.e., derived from a polymer with a certain molecular weight, or referred to as derived from a finished polymer, meaning that the polymer already exists as a polymer at the time of linking to the microsphere, rather than being formed by polymerization of monomers on the microsphere surface; because the polymer chain length is known, on one hand, polymers with the same chain length may be grafted, so that the finally formed biomicrosphere has overall uniform particle size; on the other hand, the obtained biomicrosphere has a uniform functional group density on the surface, and the adsorption capacity for biomolecules is relatively stable, i.e., the adsorption capacity of equal volumes or equal numbers of microspheres is substantially stable, thereby facilitating adjustment and optimization of parameter conditions during actual purification operations, and the systematic error generated is small. In addition, the use of a linear polymer can not only avoid a high retention ratio caused by a conventional network structure, but also be more conducive to elution after purification, thereby improving purification efficiency and precision.
(4) In the biomicrosphere of the present disclosure, the specific binding site used may be linked to the polymer on the outer surface of the biomicrosphere in the form of an affinity complex through a non-covalent strong binding force; when the purification media needs to be updated or replaced, the purification media can be conveniently and rapidly eluted from the microsphere and a new purification media can be re-bound, quickly restoring the purification performance of the glass microsphere, so that the glass microsphere may be regenerated and reused multiple times, thereby reducing the cost of separation and purification.
(5) Through the method of the present disclosure, the biomicrosphere can be prepared on a large scale; because the surface of the biomicrosphere is bound with a polymer with a fixed degree of polymerization, and the polymer is directly linked to the inorganic material through chemical reaction, the process conditions of the reaction are stable, thus overcoming the problems of unstable process conditions, high reaction difficulty, etc. existing in the method of initiating a chain polymerization reaction on the microsphere surface, which is more conducive to industrial scale-up production. Moreover, the biomicrosphere body selected in the present disclosure also features a large particle size and excellent operability, therefore, the preparation of a relatively large quantity of biomicrospheres may be realized in a single batch.
(6) Compared with existing methods in the prior art, the preparation method for the biomicrosphere of the present disclosure reduces the reaction steps for preparing the biomicrosphere, and also reduces steps such as purification, classification and fractionation; the raw materials involved (such as polyacrylic acid/sodium polyacrylate) are inexpensive and readily available; the reaction does not require an inert gas atmosphere for protection, the process is mild, and the requirements for equipment are low, thereby providing a significant overall cost advantage.
(7) In the preparation method for the biomicrosphere of the present disclosure, the type of polymer to be bound, the backbone length, etc. may also be selected according to the material and particle size of the stationary phase, the characteristics of the protein actually to be separated, the production volume demand, etc., and the adjustability in terms of the combination of the stationary phase (pressure resistance) selection, polymer type, and chromatography column loading capacity is high, thereby making the method adaptable to a broader range of production needs such as purification and separation.
(8) This biomicrosphere and the corresponding preparation method therefor not only facilitate industrial scale-up production, but also enable quantitative characterization of the unit structure, molecular weight, and topological structure of the chain, and component ratio of the copolymer, which is of great significance for the process stability and technological iteration of large-scale industrial production.

It should be understood that, within the scope of the present disclosure, the above respective technical features of the present disclosure and the respective technical features specifically described hereinafter (such as in the examples) may all be combined with one another, thereby constituting new or preferred technical solutions. Due to space limitations, they are not exhaustively enumerated herein.

### BRIEF DESCRIPTION OF THE DRAWINGS

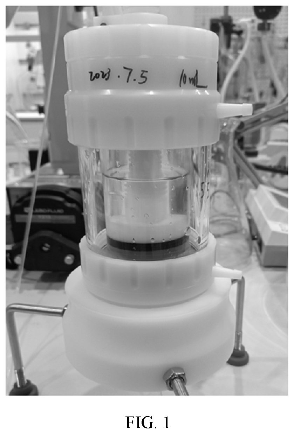

FIG. 1 shows a chromatography column after packing with the biomicrosphere.

### DETAILED DESCRIPTION OF PREFERRED EMBODIMENTS

Through extensive and intensive research, and through a large amount of screening and exploration, the present disclosure has for the first time proposed a production method for a biomicrosphere capable of large-scale preparation and use. The method directly links a polymer with a known degree of polymerization to the microsphere body, thereby improving the stability of the preparation process, and at the same time, obtaining a biomicrosphere with uniform particle size and protein loading capacity, which facilitates subsequent purification and utilization.

The present disclosure is further illustrated below in conjunction with specific embodiments and examples. For the specific methods or materials used in the examples, those skilled in the art can, on the basis of the technical concept of the present disclosure, make conventional substitutions and selections according to the existing technology, and are not limited only to the specific descriptions in the examples of the present disclosure.

### TERMINOLOGY INTRODUCTION

The "microsphere" described in the present disclosure, also referred to as microbead, microparticle, microgranule, etc., refers to micron-scale particles having an average particle size of 1 µm to 1000 µm. The particles are not limited to spherical shapes, and non-spherical shapes are permitted, for example, the shape may be ellipsoidal, polyhedral, columnar, an irregular shape, etc. Preferably, for a non-spherical shape, the ratio of the longest particle size to the smallest particle size of the particles does not exceed 5; more preferably, the ratio of the longest particle size to the smallest particle size of the particles does not exceed 2; most preferably, the particles are spherical.

In the present disclosure, "particle size" and "diameter" are involved, and when considering irregular shapes of the microsphere, since the diameters in different dimensions are different, the diameter of a regular sphere having an equivalent volume is taken as the diameter of the microspheres with irregular shapes. The "polymer" described in the present disclosure broadly includes oligomers and high polymers, having at least three structural units or a molecular weight of at least 500 Da (the molecular weight can be determined by suitable characterization methods, such as number-average molecular weight, weight-average molecular weight, viscosity-average molecular weight, etc.).

The "polymer" described in the present disclosure may be a homopolymer formed by the polymerization of a single monomer, or may be a copolymer formed by the co-reaction and polymerization of two or more monomers.

In addition, when the polymer is linked to the biomicrosphere body, a segment of a polymer with a corresponding molecular weight may be linked to the surface of the microsphere body, or through two or more reactions, two or more segments of polymers with known molecular weights may be linked to the surface of the microsphere, i.e., the polymer is linked in a manner of a plurality of segments joined together.

The acrylic polymer described in the present disclosure refers to a homopolymer or copolymer having a -C(COO-)-C- unit structure, and the copolymerization form of the copolymer is not particularly limited, as long as it can provide a linear backbone and an appropriate or stoichiometric amount of side COO- groups; the linear backbone of the acrylic polymer is permitted to contain heteroatoms. Other substituents are also permitted on the carbon-carbon double bond, as long as the progress of the polymerization reaction is not affected, for example, a methyl substituent (corresponding to -CH3C(COO-)-C-), wherein COO- may exist in the form of -COOH, may also be in a salt form (such as sodium salt), and may also be in a formate ester form (preferably an alkyl formate, e.g., carboxyl formate - COOCH3, ethyl formate -COOCH2CH3; and may also be hydroxyethyl formate - COOCH2CH2OH), etc. The specific structural forms of the -C(COO-)-C- unit structure include, but are not limited to, any one of -CH(COOH)-CH2-, -CH(COONa)-CH2-, - MeC(COOH)-CH2-, -MeC(COONa)-CH2-, -CH(COOCH3)-CH2-, -CH(COOCH2CH2OH)-CH2-, -MeC(COOCH3)-CH2-, -MeC(COOCH2CH2OH)-CH2-, etc., or any combination thereof, wherein, Me is methyl. The linear backbone of a polymer molecule may comprise only a single type of the aforementioned unit structures (corresponding to a homopolymer), or may comprise two or more types of the aforementioned unit structures (corresponding to a copolymer). After functional modification of the side carboxyl of the acrylic polymer, the - C(COO-)-C- unit structure typically takes the form of -C(CO-)-C- to form a covalent bond with an adjacent group, such as forming a linkage mode of an amide bond, an ester bond, etc., preferably forming an amide bond.

The acrylic monomer molecule described in the present disclosure refers to a monomer molecule that may be used to synthesize the aforementioned acrylic polymer, and has the basic structure of C(COO-)=C, for example, CH(COOH)=CH2, CH(COONa)=CH2, CH3C(COOH)=CH2, CH3C(COONa)=CH2, CH(COOCH3)=CH2, CH(COOCH2CH2OH)=CH2, CH3C(COOCH3)=CH2, CH3C(COOCH2CH2OH)=CH2, etc.

The linear backbone, linear-type backbone, linear-form backbone, and linear-like backbone described in the present disclosure have the same meaning and may be used interchangeably.

The branched chain described in the present disclosure refers to a chain linked to a branching point and having an independent end. In the present disclosure, the branched chain and the side branched chain have the same meaning and may be used interchangeably. In the present disclosure, the branched chain refers to a side chain or side group bonded on the linear backbone of the polymer, and there is no particular requirement for the length and size of the branched chain, which may be a short branched chain such as carboxyl, hydroxyl, amino, etc., or may be a long branched chain containing a relatively large number of atoms. There is no particular requirement for the structure of the branched chain, which may be linear or may be a branched chain having a branching structure. The branched chain may also contain additional side chains or side groups. The quantity, length, size, degree of re-branching, and other structural characteristics of the branched chains are preferably such that a network structure is not formed as much as possible, and branched chain accumulation is not caused to the extent that the retention ratio increases, at which point the flexible swinging of the linear backbone may be smoothly exerted.

In the present disclosure, the "fixation" mode such as fixed, fixed to, fixed with, fixed on, etc., refer to a covalent binding mode.

In the present disclosure, the "connection"/"binding" mode such as carrying, linked with, linked to, linked on, binding, capturing, captured onto, etc., are not particularly limited, and include, but are not limited to, covalent modes, non-covalent modes, and other modes.

The covalent mode described in the present disclosure refers to a mode of direct bonding via a covalent bond. The covalent mode includes, but is not limited to, a dynamic covalent mode, and the dynamic covalent mode refers to a mode of direct bonding via a dynamic covalent bond.

The covalent bond described in the present disclosure includes common covalent bonds such as an amide bond, an ester bond, etc., and also includes dynamic covalent bonds having reversible properties. The covalent bond includes a dynamic covalent bond. A dynamic covalent bond is a chemical bond having reversible properties, including, but not limited to, an imine bond, an acylhydrazone bond, a disulfide bond, or a combination thereof, and those skilled in the field of chemistry may understand the meaning of the dynamic covalent bond.

The non-covalent mode described in the present disclosure includes, but is not limited to, supramolecular interaction modes such as coordination binding, affinity complex interaction, electrostatic adsorption, hydrogen bond, π-π stacking interaction, hydrophobic interaction, etc.

The supramolecular interaction described in the present disclosure includes, but is not limited to, coordination binding, affinity complex interaction, electrostatic adsorption, hydrogen bond, π-π stacking interaction, hydrophobic interaction, and a combination thereof.

The functional group refers to a group that is located on the polymer that has the ability to attach, bind, etc. to other groups or molecules, etc., and is for example hydroxyl, carboxyl, amino, sulfhydryl, etc., and combinations of the above groups.

The specific binding site described in the present disclosure refers to a group or structural site possessing a specific binding function, the group or structural site possesses the function of specific recognition and binding to a certain specific target, the specific binding can be achieved through binding effects such as coordination, complexation, electrostatic force, Van der Waals force, hydrogen bond, covalent bond, etc. or other interactions.

The biological target described in the present disclosure, or referred to as target, also referred to as purification substrate, is a substance to be separated from a mixed system. The purification substrate in the present disclosure is not particularly limited; preferably, the purification substrate is a proteinaceous substance (also referred to as target protein in this case), a polypeptide, a nucleic acid, a cell, a polysaccharide, etc.

The purification media described in the present disclosure belongs to one type of the specific binding site, and refers to a substance capable of specifically binding to the purification substrate, thereby capturing the purification substrate, and further capable of separating the purification substrate from a mixed system. The purification media linked to the functional group of the polymer of the present disclosure is a functional element having the function of binding the purification substrate. When the purification media is linked to an adjacent group in a covalent mode, it typically serves as a group having the function of binding the purification substrate.

The affinity protein described in the present disclosure specifically binds to a target protein and has relatively high affinity binding force, and is for example, Protein A, Protein G, Protein L, modified Protein A, modified Protein G, modified Protein L, etc.

The biotin described in the present disclosure is capable of binding to avidin, and has strong binding force and good specificity.

The avidin described in the present disclosure is capable of binding to biotin, and has strong binding force and good specificity, and is for example streptavidin (abbreviated as SA), including protein subunits, analogs (such as Tamvavidin2, abbreviated as Tam2), modified products and mutants thereof, and the like.

The biotin analog described in the present disclosure refers to a non-biotin molecule capable of forming "avidin-biotin"-like specific binding with avidin, preferably one such biotin analog is a polypeptide or a protein, for example, a polypeptide containing the WSHPQFEK sequence used in the Strep-tag^{®} series developed by IBA company (such as Strep^{®}II, Twin-Strep-tag^{®} , etc.), and similar polypeptides containing the WNHPQFEK sequence. WNHPQFEK may be regarded as a mutant sequence of WSHPQFEK.

The avidin analog described in the present disclosure refers to a non-avidin molecule capable of forming "avidin-biotin"-like specific binding with biotin, preferably one such biotin analog is a polypeptide or a protein. The avidin analog includes, but is not limited to, derivatives of avidin, homologous substances (homologs) of avidin, variants of avidin, etc. The avidin analog is for example, Tamavidin1, Tamavidin2, etc. (reference may be made to: FEBS Journal, 2009, 276, 1383-1397).

In the present disclosure, "biotin or biotin analog" and "biotin or analog thereof" have the same meaning and may be used interchangeably.

In the present disclosure, "avidin or avidin analog" and "avidin or analog thereof" have the same meaning and may be used interchangeably.

Biotin-type tag: the biotin-type tag contains the following units: biotin, an avidin analog capable of binding avidin, an avidin analog capable of binding an avidin analog, and a combination thereof. The biotin-type tag can specifically bind to avidin, an avidin analog, or a combination thereof. Therefore, the biotin-type tag may be used to separate and purify, including but not limited to, proteinaceous substances labeled with an avidin-type tag.

The avidin-type tag described in the present disclosure: the avidin-type tag contains the following units: avidin, an avidin analog capable of binding biotin, an avidin analog capable of binding a biotin analog, and a combination thereof. The avidin-type tag can specifically bind to biotin, a biotin analog, or a combination thereof. Therefore, the avidin-type tag may be used to separate and purify, including but not limited to, proteinaceous substances labeled with a biotin-type tag.

The polypeptide-type tag described in the present disclosure refers to a tag containing a polypeptide tag or a derivative of a polypeptide tag. The polypeptide tag refers to a tag of a polypeptide structure composed of amino acid units, and the amino acids may be natural amino acids or may be unnatural amino acids.

The protein-type tag described in the present disclosure includes a tag containing a protein tag or a derivative of a protein tag. The protein tag refers to a tag of a protein structure composed of amino acid units, and the amino acids may be natural amino acids or may be unnatural amino acids.

The antibody-type tag described in the present disclosure refers to a tag containing an antibody-like substance, which can specifically bind to a corresponding target, such as an antigen. Examples of the antibody-type tag also include anti-eGFP nanobody capable of specifically binding to eGFP protein.

The antigen-type tag described in the present disclosure refers to a tag containing an antigen-like substance, which can specifically bind to an antibody-like substance.

The peptide described in the present disclosure is a compound in which two or more amino acids are linked by peptide bonds. In the present disclosure, peptide and peptide segment have the same meaning and may be used interchangeably.

The polypeptide described in the present disclosure refers to a peptide composed of 10 to 50 amino acids.

The protein described in the present disclosure refers to a peptide composed of 50 or more amino acids. A fusion protein is also a protein.

The proteinaceous substance described in the present disclosure broadly refers to a substance containing a polypeptide or a protein fragment. For example, a polypeptide derivative, a protein derivative, a glycoprotein, etc. are also included within the scope of proteinaceous substances.

The binding force described in the present disclosure refers to binding ability, such as the binding ability of a biomicrosphere to a certain protein.

KH570: 3-(methacryloyloxy)propyltrimethoxysilane, also referred to as gamma-methacryloyloxypropyltrimethoxysilane, CAS: 2530-85-0, an acryloyl-functionalized silane coupling agent.

KH550: 3-aminopropyltriethoxysilane, CAS: 919-30-2, an aminated silane coupling agent. The molecular formula is NH2-(CH2)3-Si(OCH2CH3)3.

KH590: gamma-mercaptopropyltrimethoxysilane, CAS: 4420-74-0, a silane coupling agent having a sulfhydryl functional group, with the molecular formula C6H16O3SSi.

NTA: nitrilotriacetic acid, also referred to as nitrilotriacetic acid. In some positions in the present disclosure, NTA refers to the corresponding residue.

The biomicrosphere described in the present disclosure is specifically characterized in that the outer surface of the microsphere body of the biomicrosphere is covalently coupled with at least one polymer chain, and other portions of the polymer chain are unattached to the outer surface of the microsphere body; the polymer chain contains a functional group, and the functional group specifically binds to a biological target via a specific site, wherein the polymer chain comprises at least one segment, each of the at least one segment is derived from a polymer, and the at least one segment of the polymer chain is derived from a polymer having a molecular weight > 5000; the particle size of the biomicrosphere body is ≥ 10 µm, wherein:
the outer surface of the microsphere body of the biomicrosphere is covalently coupled with at least one polymer chain: the "at least one" herein means that the polymer chains have different molecular weights or constituent monomers and belong to different types;
"the other portions of the polymer chain are unattached to the outer surface of the microsphere body" refers to portions other than the portion covalently coupled to the surface of the microsphere body;
the polymer chain comprises at least one segment, each segment is derived from a polymer: when the polymer chain comprises one segment, this means that the polymer chain is derived from one existing polymer; when the polymer chain comprises a plurality of segments, this means that the polymer chain comprises two portions, each portion is derived from an existing polymer, and these polymers are linked to one another. The biomicrosphere refers to a microsphere having a purification function that can specifically adsorb proteins, polypeptides, or other biomolecules to be separated, i.e., biological targets, thereby separating the biological target molecules from the mixture. The biomicrosphere body is made of an inorganic or organic material, and the surface thereof is grafted with a macromolecular polymer having functional groups to recognize and bind to the target substance.

The "derived from a polymer" herein means that a polymer with known degree of polymerization is used, i.e., the polymer is derived from a polymer with a certain molecular weight, or referred to as derived from a finished polymer, meaning that an already obtained polymer is used, rather than being formed by polymerization of a monomer on the microsphere surface.

The polymer chain is derived from a polymer, i.e., the molecular weight of the polymer is clearly known before the binding reaction, and it may be the same polymer with the same molecular weight, or may be a combination of known polymers with different molecular weights. If a pure polymer having the same molecular weight is used for binding to the biomicrosphere body, a biomicrosphere having a uniform surface linear length can be obtained. Moreover, when a linear polymer is used, no crosslinking agent is used during the binding or grafting reaction, thereby ensuring that the linear polymer can still exist in a linear form, and no cross-linking phenomenon occurs between the chains, no network is formed, thereby avoiding retention of macromolecular substances caused by network pores. The biomicrosphere body (or referred to as microsphere body) has a particle size relatively larger than that of commonly used microspheres, i.e., an average particle size of ≥ 10 µm, and the selection of a relatively large particle size facilitates the grafting reaction and operations such as loading and unloading of the packed column in subsequent purification, and because the gaps between the particles are relatively large, the retention or clogging of macromolecules can also be effectively reduced. Matching the large-particle-size biomicrosphere body, the molecular weight of the linear polymer is also selected to be relatively large, such as a molecular weight > 5000, and by increasing the chain length, the number of specific sites for binding to proteins and other macromolecules may be increased, thereby overcoming the problem of reduced specific surface area caused by the increase in the size of the biomicrosphere body, and thus maintaining the adsorption capacity for protein molecules.

In one example,
the functional group of the polymer is one of carboxyl, hydroxyl, amino, and sulfhydryl or a combination thereof,
the shape of the polymer may be linear, comb-like, etc., as long as the polymer is a macromolecule and does not contain a large amount of network structures; considering factors such as cost and ease of reaction control, the polymer is preferably a linear polymer. The linear polymer may have or may not have a branched chain, and when the linear polymer has a branched chain, the functional group may be located at the end of the branched chain. The functional group is a group located on the macromolecular polymer capable of binding to other groups or molecules, so as to specifically bind to a target substance or target group.

The material of the microsphere body is an inorganic material or an organic material containing hydroxyl. There is no particular requirement for the material of the biomicrosphere body, and any commonly used microsphere material suitable for separation and purification or having a surface capable of linking to a polymer under modified or unmodified conditions is acceptable; common microsphere body materials may be selected from any one of or a combination of glass, agarose, magnetic material, silica, and hydroxyl-containing high molecular polymer, preferably a material whose surface is glass or silica. For the morphology of the microsphere body, as described above, it is not limited to a spherical shape, and non-spherical shapes are permitted, for example, the shape may be ellipsoidal, polyhedral, columnar, an irregular shape, etc.; the microsphere body may be solid, or may have a cavity, and the number of cavities is not limited; that is to say, the microsphere mainly plays the adsorption role through grafting of high molecular polymers on the surface, and therefore, the internal structure of the microsphere is not particularly limited. Preferably, a solid microsphere body is used.

In one example, the monomer of the linear polymer is an acrylic polymer, an acrylic copolymer, an amino acid polymer, or other carboxylic acid polymers. Preferably, the monomer unit of the acrylic polymer comprises one of acrylic acid, acrylate salt, acrylate ester, methacrylic acid, methacrylate salt, and methacrylate ester or a derivative thereof, or any combination of the above, and the polymer is obtained by polymerizing the above monomers or combinations thereof; preferably, no crosslinking agent is needed during the polymerization process, thereby forming a linear polymer. In addition to polymers formed by the polymerization of a single monomer, the polymer also includes copolymers of different monomers, and any linear polymer that satisfies the condition of having functional groups on the branched chain and being capable of binding to the biomicrosphere body may be selected, such as a copolymer of 3-(methacryloyloxy)propyltrimethoxysilane and N-hydroxysuccinimide acrylate, etc.

In one example, the functional group contains a specific binding site, and the specific binding site can specifically bind to a target; the specific binding site comprises a metal ion, biotin, a biotin analog, avidin, an avidin analog, an antibody-type tag, or an antigenic tag, a polypeptide-type tag, a protein-type tag, an immune-type tag, or a combination thereof; the linkage mode of the specific binding site to the functional group is: a covalent bond, a supramolecular interaction, a linking element, or a combination thereof. In a preferred example, the specific binding site comprises a nickel ion, biotin, or a biotin analog. It is well known that nickel ions can specifically bind to a marker with a His-tag (histidine tag), and biotin or an analog thereof may serve either as a purification media or as a linking element for further linking other types of purification media, and the biotin or analog thereof includes desthiobiotin, etc.

In one example, the molecular weight of the polymer is: greater than or equal to 50,000, greater than or equal to 150,000, 450,000-6,000,000, or 450,000-4,500,000, preferably, the molecular weight of the linear polymer is 50,000, 150,000, 200,000, 300,000, 400,000, 500,000, 600,000, 700,000, 800,000, 900,000, 1,000,000, 1,250,000, 1,500,000, 1,800,000, 2,000,000, 2,500,000, 3,000,000, 3,500,000, 4,000,000, 4,500,000, 5,000,000, or 6,000,000. Using a larger molecular weight, i.e., a polymer of longer chain length, provides more branched chains and functional groups, thereby increasing the sites for binding to the target substance and improving the overall loading capacity of the biomicrosphere.

In another example, the particle size of the biomicrosphere body is 10 µm-600 µm, 30 µm-600 µm, greater than or equal to 30 µm and less than 600 µm, preferably, the particle size of the biomicrosphere body is 10 µm, 40 µm, 50 µm, 60 µm, 70 µm, 80 µm, 90 µm, 100 µm, 120 µm, 150 µm, 200 µm, 300 µm, 400 µm, and 600 µm. Compared with commonly used biomicrospheres in the past, the present disclosure employs a microsphere body of larger particle size to facilitate large-scale production and preparation, and to avoid the retention phenomenon of biomolecules.

In another example, the biomicrosphere has no network cross-linking. Having no network cross-linking can better avoid the obstruction of the flow of biological macromolecules caused by the network pore structure, preventing incomplete separation caused by macromolecule retention.

In another example, the linear polymer is a polymer formed from acrylic acid or sodium acrylate monomers, with a molecular weight of 4,500,000, and the particle size of the biomicrosphere body is 100 µm. As a more commonly used linear polymer, polyacrylic acid or sodium polyacrylate is inexpensive and readily available, and easy to operate; more importantly, the branched chains of the linear polymer contain a large number of functional groups, and thus polyacrylic acid or sodium polyacrylate is a preferred choice. When polyacrylic acid or sodium polyacrylate having a molecular weight of 4,500,000 bound to a biomicrosphere body having a particle size of 100 µm is used, upon testing, the protein adsorption effect is good; compared with the small-particle biomicrospheres in the prior art, the cost is greatly reduced, and the operability is improved.

In another example, the biomicrosphere is obtained by coupling the polymer to the outer surface of the microsphere body. The coupling of the polymer chain to the surface of the microsphere body is achieved by covalently coupling the polymer which is known to the outer surface of the microsphere body;
for a polymer chain having a plurality of the segments, one known polymer is covalently coupled to the outer surface of the microsphere body and then linked to another known polymer.

The coupling may be completed in one step, or may be completed in two or more steps; however, the two or more steps herein do not include a polymerization reaction, but merely refer to stepwise grafting of macromolecules, thereby increasing the chain length of the overall polymer to a certain extent.

In another example, the biomicrosphere further has any one or more of the following features:
(1) when the polymer chain comprises a plurality of segments, the polymer from which each segment is derived is the same or different;
(2) the microsphere body is a solid microsphere;
(3) the microsphere body is a glass microsphere or a microsphere containing a magnetic material. "The polymer comprises a plurality of segments" may refer to, through a stepwise reaction, first grafting a segment of a polymer with a known molecular weight onto the surface of the biomicrosphere, and then grafting another known polymer, thereby increasing the chain length to a certain extent. The polymers added in each reaction step may be the same or different: "same" means that both the size and the constituent monomers are the same.

The present disclosure further provides a preparation method for a biomicrosphere, which is obtained by coupling a polymer to the surface of the microsphere body, i.e., the coupling of the polymer chain to the surface of the microsphere body is achieved by covalently coupling the polymer which is known to the outer surface of the microsphere body;
for a polymer chain having a plurality of the segments, one known polymer is covalently coupled to the outer surface of the microsphere body and then linked to another known polymer.

Preferably, no crosslinking agent is used and/or the polymer is a linear polymer. Through a mild coupling reaction, the macromolecular polymer may be directly linked to the biomicrosphere body.

Specifically, in one example, the preparation method comprises the following steps:
(1) subjecting the biomicrosphere body to chemical modification, introducing amino or other groups capable of binding to carboxyl onto the outer surface of the biomicrosphere body, to form a biomicrosphere A;
(2) under the condition of no crosslinking agent, linking a polymer to a surface of the biomicrosphere A to obtain a biomicrosphere B.

Preferably, in step (1), the biomicrosphere body is chemically modified using a silane coupling agent; preferably, an aminated silane coupling agent is used. Commonly used silane coupling agents include, for example: gamma-mercaptopropyltrimethoxysilane, 3-aminopropyltriethoxysilane, 3-(methacryloyloxy)propyltrimethoxysilane, etc. The hydroxyl and other groups on the surface of the biomicrosphere body are used to react with the silane coupling agent, so that the surface of the biomicrosphere body is modified, thereby enabling further binding to a polymer. Thereafter, the polymer is bound to the modified biomicrosphere under mild conditions, thereby obtaining a biomicrosphere having a macromolecular polymer linked on the surface and possessing branched chains. The above reaction does not require the addition of a crosslinking agent and does not produce a network structure, thereby ensuring the uniformity of the structure of the polymer, especially the linear polymer.

The preparation method for the biomicrosphere may further employ the following steps:
linking the silane coupling agent to the linear polymer, and then linking the resulting product to the biomicrosphere body to obtain a biomicrosphere B.

In another example, the step (1) is carried out under heating conditions, optionally at 40-100°C, for a reaction time of 24-72 hours; or reacting at 50-80°C for 36-48 hours; a specific example is: reacting at 60°C for 48 hours.

In another example, the step (2) is carried out under mild conditions, for example, reacting at 25-40°C for 1-5 hours, and a specific example is reacting at 37°C for 2 hours. The mild conditions employed in this reaction not only improve the operability and the reliability of large-scale production, but also reduce the cost.

In another example, the preparation method for the biomicrosphere further comprises: (3) under the condition of no crosslinking agent, further linking other polymers to the surface of the biomicrosphere B to obtain a biomicrosphere D. Different polymers may be flexibly combined according to actual demands, thereby obtaining chains of different lengths, or other chains that meet actual demands.

In another example, the above-mentioned preparation method for the biomicrosphere further comprises the following steps: carrying out a reaction of linking a functional group of the polymer to a specific binding site, preferably, using the biomicrosphere B or the biomicrosphere D to carry out the reaction of linking the functional group of the polymer to the specific binding site.

By linking a specific binding site, the function of specifically binding the target substance may be exerted. In different linkage modes, the binding employed may be covalent or non-covalent, for example, binding may be achieved through modes of action such as coordination, complexation, electrostatic force, Van der Waals force, hydrogen bond, covalent bond, etc. or other modes of action.

In another example, the above-mentioned preparation method for the biomicrosphere further comprises the following steps: coupling the biomicrosphere B or the biomicrosphere D with tricarboxyamine, and then complexing the resulting product with Ni ions to obtain a biomicrosphere C;
or, linking biotin, a biotin analog, avidin, an avidin analog, an antibody-type tag, or an antigenic tag to the functional group;
preferably, the tricarboxyamine is N,N-bis(carboxymethyl)-L-lysine, nitrilotriacetic acid, and a combination thereof.

Preferably, the Ni ions may be provided in the form of nickel sulfate, etc.

Preferably, the reaction of coupling the biomicrosphere B or the biomicrosphere D with tricarboxyamine is carried out at 25-40°C for 1-5 hours, preferably at 37°C for 2 hours. The biomicrosphere after complexing nickel ions can bind to a His-tag, thereby separating the target substance containing the His-tag.

In another example, in the above-mentioned large-scale preparation method for the biomicrosphere, the ratio of the usage amount of the polymer to the biomicrosphere body is: 8 × 10⁻⁷ mol/10 mL-2.0 × 10⁻⁵ mol/10 mL, optionally, 1.2 × 10⁻⁶ mol/10 mL, 1.3 × 10⁻⁶ mol/10 mL, 1.5 × 10⁻⁶ mol/10 mL, 2.0 × 10⁻⁶ mol/10 mL, mol/10 mL refers to the number of moles of the polymer used per 10 mL of microspheres, and for a polymer chain having a plurality of the segments, the calculation is based on the first segment of the polymer. The biomicrosphere body herein may be a microsphere that has not undergone surface treatment, or may refer to a microsphere after surface modification treatment; because only the surface is modified, the diameter does not change significantly, and the volume also does not change significantly. The polymer is preferably a linear polymer. Compared with common biomicrospheres, the usage amount of the linear polymer is reduced, and the cost is reduced; however, because the chain length of the linear polymer is greatly increased, the overall adsorption capacity of the biomicrosphere can be ensured.

In another example, a method for separating a biological target is further provided, and the main feature of the method is using the aforementioned biomicrosphere and binding the biomicrosphere to the biological target.

Specifically, the method comprises the following steps:
(1) packing the biomicrosphere of the aforementioned example into a chromatography column;
(2) injecting the solution containing the biological target to be separated into the chromatography column for adsorption;
(3) eluting the chromatography column and collecting the biological target.
the biological target is one or more of a protein, a nucleic acid, a cell, a polysaccharide, and a peptide.

According to the properties of the biological target substance to be separated (such as protein molecules), such as molecular size, the tag carried, specific groups, etc., a biomicrosphere with a suitable particle size and degree of polymerization is selected, or the biomicrosphere to be used is prepared according to actual separation demands, and the usage amount of the biomicrosphere is adjusted and selected according to the volume of the solution containing the target substance to be tested, and the microsphere is packed into a chromatography column. Typically, the volume ratio of the biomagnetic microsphere to the solution containing the target substance to be treated is 1:10-1:80, preferably 1:10-1:60, and more preferably 1:20-1:40; the specific usage amount is adjusted and optimized according to the content of the target substance actually present in the solution. The above adsorption and elution methods may be carried out with reference to conventional parameters and conditions in the art.

In another example, a separation system is further provided, wherein the separation system uses the biomicrosphere of the aforementioned example, or uses the biomicrosphere prepared by the method of the aforementioned example. The biomicrosphere has the characteristics of a large particle size, a large molecular weight of the polymer grafted on the surface, and a large number of specific adsorption sites possessed, etc.; because the biomicrosphere of the present disclosure is used, the operability of the protein purification system is enhanced, making the system adaptable to large-scale production, and the cost of separation is reduced.

The detailed methods of the present disclosure are explained below using specific examples.

### Example 1

(1) Several glass beads having a diameter of 100 µm were weighed and placed into a 2,000 mL beaker, after ultrasonic soaking with 1 M sodium hydroxide, the beaker was maintained in a 70-degree Celsius water bath for 90 min, the glass beads were ultrasonically washed three times with deionized water until neutral (pH test paper), and finally subjected to displacement washing once with anhydrous ethanol.
(2) 1 L of the washed glass beads was taken; after ethanol displacement was performed (3 times), the obtained glass beads were transferred to a four-necked reaction vessel; after the paste-like glass beads were washed with ethanol and transferred, the ethanol was controlled at 5 L; the stirrer was turned on, and the stirring speed was adjusted to about 180 rpm; the temperature was raised to 60°C, 0.5 L of 3-aminopropyltriethoxysilane was added, the reaction was started, and the reaction was continued for 48 h; after completion of the reaction, washing was performed alternately with three times the volume of ethanol and water until the pH was neutral.
(3) 10 mL of the glass beads modified with 3-aminopropyltriethoxysilane were re-dispersed in 30 mL of 2-morpholinoethanesulfonic acid solution (pH = 5.0), and 0.59 g of polyacrylic acid having a molecular weight of 450,000, 1.05 g of 1-ethyl-3-(3-dimethylaminopropyl)carbodiimide hydrochloride, and 0.73 g of N-hydroxysuccinimide were added, and the reaction was carried out at 37°C for 2 h. After completion of the reaction, washing was performed 4 times with 2-morpholinoethanesulfonic acid solution.
(4) 5 mL of the washed glass beads were re-dispersed in 30 mL of 2-morpholinoethanesulfonic acid solution (pH = 5.0), and 5.32 g of nitrilotriacetic acid disodium salt, 1.31 g of 1-ethyl-3-(3-dimethylaminopropyl)carbodiimide hydrochloride, and 0.91 g of N-hydroxysuccinimide were added, and the reaction was carried out at 37°C for 2 h.
(5) After completion of the reaction, washing was repeated 3 times with 2-morpholinoethanesulfonic acid solution, the washed glass beads were immersed in 25 mL of 100 mM nickel sulfate solution, and the reaction was carried out with stirring at room temperature for 1 hour, to obtain a biomicrosphere containing nickel ions.

Effect test: After the reaction was completed, the glass beads were packed into a column, the protein loading capacity of the glass beads was tested using a histidine-tagged protein, and the protein loading capacity of the batch of glass beads was measured to be 1.1 mg/mL.

### Example 2

Step (3) was performed according to the following operation, and the remaining steps were the same as those in Example 1.

(3) 10 mL of the glass beads modified with 3-aminopropyltriethoxysilane were re-dispersed in 60 mL of 2-morpholinoethanesulfonic acid solution (pH = 5.0), and 1.64 g of polyacrylic acid having a molecular weight of 1,250,000, 2.92 g of 1-ethyl-3-(3-dimethylaminopropyl)carbodiimide hydrochloride, and 2.03 g of N-hydroxysuccinimide were added, and the reaction was carried out at 37°C for 2 h. After completion of the reaction, washing was performed 4 times with 2-morpholinoethanesulfonic acid solution.

Effect test: After the reaction was completed, the glass beads were packed into a column, the protein loading capacity of the glass beads was tested using a histidine-tagged protein, and the protein loading capacity of the batch of glass beads was measured to be 3.6 mg/mL.

### Example 3

Step (3) was performed according to the following operation, and the remaining steps were the same as those in Example 1.

(3) 10 mL of the glass beads modified with 3-aminopropyltriethoxysilane were re-dispersed in 60 mL of 2-morpholinoethanesulfonic acid solution (pH = 6.0), and 2.36 g of sodium polyacrylate having a molecular weight of 4,500,000, 2.35 g of 1-ethyl-3-(3-dimethylaminopropyl)carbodiimide hydrochloride, and 1.64 g of N-hydroxysuccinimide were added, and the reaction was carried out at 37°C for 2 h. After completion of the reaction, washing was performed 4 times with 2-morpholinoethanesulfonic acid solution.

Effect test: After the reaction was completed, the glass beads were packed into a column, the protein loading capacity of the glass beads was tested using a histidine-tagged protein, and the protein loading capacity of the batch of glass beads was measured to be 9.5 mg/mL.

### Example 4

Step (3) was performed according to the following operation, and the remaining steps were the same as those in Example 1.

(3) 10 mL of the glass beads modified with 3-aminopropyltriethoxysilane were re-dispersed in 60 mL of 2-morpholinoethanesulfonic acid solution (pH = 6.0), and 0.06 g of polyacrylic acid having a molecular weight of 5,000, 1.2 g of 1-ethyl-3-(3-dimethylaminopropyl)carbodiimide hydrochloride, and 0.84 g of N-hydroxysuccinimide were added, and the reaction was carried out at 37°C for 2 h. After completion of the reaction, washing was performed 4 times with 2-morpholinoethanesulfonic acid solution.

Effect test: After the reaction was completed, the glass beads were packed into a column, the protein loading capacity of the glass beads was tested using a histidine-tagged protein, and the protein loading capacity of the batch of glass beads was measured to be 0.1 mg/mL.

### Example 5

(1) Several glass beads having a diameter of 40 µm were weighed and placed into a 2,000 mL beaker, after ultrasonic soaking with 1 M sodium hydroxide, the beaker was maintained in a 70-degree Celsius water bath for 90 min, the glass beads were ultrasonically washed three times with deionized water until neutral (pH test paper), and finally subjected to displacement washing once with anhydrous ethanol.
(2) 1 L of the washed glass beads was taken; after ethanol displacement was performed (3 times), the obtained glass beads were transferred to a four-necked reaction vessel; after the paste-like glass beads were washed with ethanol and transferred, the ethanol was controlled at 5 L; the stirrer was turned on, and the stirring speed was adjusted to about 180 rpm; the temperature was raised to 60°C, 0.5 L of 3-aminopropyltriethoxysilane was added, the reaction was started, and the reaction was continued for 48 h; after completion of the reaction, washing was performed alternately with three times the volume of ethanol and water until the pH was neutral.
(3) 10 mL of the glass beads modified with 3-aminopropyltriethoxysilane were re-dispersed in 20 mL of 2-morpholinoethanesulfonic acid solution (pH = 4.1), and 0.21 g of sodium polyacrylate having a molecular weight of 150,000, 1.05 g of 1-ethyl-3-(3-dimethylaminopropyl)carbodiimide hydrochloride, and 1.46 g of N-hydroxysuccinimide were added, and the reaction was carried out at 30°C for 2 h. After completion of the reaction, washing was performed 4 times with 2-morpholinoethanesulfonic acid solution.
(4) 5 mL of the washed glass beads were re-dispersed in 30 mL of 2-morpholinoethanesulfonic acid solution (pH = 5.0), and 5.32 g of nitrilotriacetic acid disodium salt, 1.31 g of 1-ethyl-3-(3-dimethylaminopropyl)carbodiimide hydrochloride, and 0.91 g of N-hydroxysuccinimide were added, and the reaction was carried out at 25°C for 12 h.
(5) After completion of the reaction, washing was repeated 3 times with 2-morpholinoethanesulfonic acid solution, the washed glass beads were immersed in 25 mL of 100 mM nickel sulfate solution, and the reaction was carried out with stirring at room temperature for 1 hour.

Effect test: After the reaction was completed, the glass beads were packed into a column, the protein loading capacity of the glass beads was tested using a histidine-tagged protein, and the protein loading capacity of the batch of glass beads was measured to be 4.5 mg/mL.

### Example 6

(1) Several glass beads having a diameter of 400 µm were weighed and placed into a 2,000 mL beaker, after ultrasonic soaking with 1 M sodium hydroxide, the beaker was maintained in a 70-degree Celsius water bath for 90 min, the glass beads were ultrasonically washed three times with deionized water until neutral (pH test paper), and finally subjected to displacement washing once with anhydrous ethanol.
(2) 1 L of the washed glass beads was taken; after ethanol displacement was performed (3 times), the obtained glass beads were transferred to a four-necked reaction vessel; after the paste-like glass beads were washed with ethanol and transferred, the ethanol was controlled at 5 L; the stirrer was turned on, and the stirring speed was adjusted to about 180 rpm; the temperature was raised to 60°C, 0.5 L of 3-aminopropyltriethoxysilane was added, the reaction was started, and the reaction was continued for 48 h; after completion of the reaction, washing was performed alternately with three times the volume of ethanol and water until the pH was neutral.
(3) 10 mL of the glass beads modified with 3-aminopropyltriethoxysilane were re-dispersed in 20 mL of 2-morpholinoethanesulfonic acid solution (pH = 3.5), and 2.36 g of polyacrylic acid having a molecular weight of 4,500,000, 2.35 g of 1-ethyl-3-(3-dimethylaminopropyl)carbodiimide hydrochloride, and 1.64 g of N-hydroxysuccinimide were added, and the reaction was carried out at 30°C for 2 h. After completion of the reaction, washing was performed 4 times with 2-morpholinoethanesulfonic acid solution.
(4) 5 mL of the washed glass beads were re-dispersed in 30 mL of 2-morpholinoethanesulfonic acid solution (pH = 5.0), and 5.32 g of nitrilotriacetic acid disodium salt, 1.31 g of 1-ethyl-3-(3-dimethylaminopropyl)carbodiimide hydrochloride, and 0.91 g of N-hydroxysuccinimide were added, and the reaction was carried out at 25°C for 12 h.
(5) After completion of the reaction, washing was repeated 3 times with 2-morpholinoethanesulfonic acid solution, the washed glass beads were immersed in 25 mL of 100 mM nickel sulfate solution, and the reaction was carried out with stirring at room temperature for 1 hour.

Effect test: After the reaction was completed, the glass beads were packed into a column, the protein loading capacity of the glass beads was tested using a histidine-tagged protein, and the protein loading capacity of the batch of glass beads was measured to be 0.9 mg/mL.

### Example 7

(1) Several glass beads having a diameter of 200 µm were weighed and placed into a 2000 mL beaker, after ultrasonic soaking with 1 M sodium hydroxide, the beaker was maintained in a 70-degree Celsius water bath for 60 min, the glass beads were ultrasonically washed three times with deionized water until neutral (pH test paper), and finally subjected to displacement washing once with anhydrous ethanol.
(2) 1 L of the washed glass beads was taken; after ethanol displacement was performed (3 times), the obtained glass beads were transferred to a four-necked reaction vessel; after the paste-like glass beads were washed with ethanol and transferred, the ethanol was controlled at 5 L; the stirrer was turned on, and the stirring speed was adjusted to about 180 rpm; the temperature was raised to 60°C, 0.5 L of 3-mercaptopropyltriethoxysilane was added, the reaction was started, and the reaction was continued for 48 h; after completion of the reaction, washing was performed alternately with three times the volume of ethanol and water until the pH was neutral.
(3) 20 mL of the glass beads modified with 3-mercaptopropyltriethoxysilane were dispersed in 120 mL of ethanol, and 0.095 g of methacrylate-polyethylene glycol (PEG)-carboxylic acid having a molecular weight of 5,000 and 0.01 g of triethylamine were added, and the reaction was carried out at room temperature for 24 hours. After completion of the reaction, washing was performed 4 times with 2-morpholinoethanesulfonic acid solution.
(4) 10 mL of the washed glass beads were re-dispersed in 60 mL of 2-morpholinoethanesulfonic acid solution (pH = 5.0), and 0.30 g of N,N-bis(carboxymethyl)-L-lysine tricarboxyamine, 2.62 g of 1-ethyl-3-(3-dimethylaminopropyl)carbodiimide hydrochloride, and 1.81 g of N-hydroxysuccinimide were added, and the reaction was carried out at 25°C for 8 h.
(5) After completion of the reaction, washing was repeated 3 times with 2-morpholinoethanesulfonic acid solution, the washed glass beads were immersed in 25 mL of 100 mM nickel sulfate solution, and the reaction was carried out with stirring at room temperature for 1 hour.

Effect test: After the reaction was completed, the glass beads were packed into a column, the protein loading capacity of the glass beads was tested using a histidine-tagged protein, and the protein loading capacity of the batch of glass beads was measured to be 0.3 mg/mL.

### Example 8

(1) Several glass beads having a diameter of 100 µm were weighed and placed into a 1,000 mL beaker, after ultrasonic soaking with 1 M sodium hydroxide, the beaker was maintained in a 70-degree Celsius water bath for 90 min, the glass beads were ultrasonically washed three times with deionized water until neutral (pH test paper), and finally subjected to displacement washing 3 times with anhydrous ethanol.
(2) 10 mL of the washed glass beads were re-suspended in 60 mL of anhydrous tetrahydrofuran and placed into a three-necked flask, and 0.30 g of a polymer of 3-(methacryloyloxy)propyltrimethoxysilane-co-N-hydroxysuccinimide acrylate having a molecular weight of 250,000 was added, and the water bath was heated to 60 degrees Celsius for reaction for 12 hours. Then, washing was performed 3 times with anhydrous tetrahydrofuran.
(3) 10 mL of the washed glass beads were re-dispersed in 30 mL of 2-morpholinoethanesulfonic acid solution (pH = 5.0), and 10.6 g of N,N-bis(carboxymethyl)-L-lysine was added, and the reaction was carried out at 25°C for 12 h.
(4) After completion of the reaction, washing was repeated 3 times with 2-morpholinoethanesulfonic acid solution, the washed glass beads were immersed in 25 mL of 100 mM nickel sulfate solution, and the reaction was carried out with stirring at room temperature for 1 hour.

Effect test: After the reaction was completed, the glass beads were packed into a column, the protein loading capacity of the glass beads was tested using a histidine-tagged protein, and the protein loading capacity of the batch of glass beads was measured to be 2.6 mg/mL.

### Example 9

(1) Several agarose beads having a diameter of 200 µm were weighed and placed into a 1,000 mL beaker, ultrasonically washed three times with deionized water, and finally subjected to displacement washing 3 times with anhydrous ethanol.
(2) 100 mL of the washed agarose beads were re-suspended in 600 mL of anhydrous tetrahydrofuran and placed into a three-necked flask, and 3.0 g of a polymer of 3-(methacryloyloxy)propyltrimethoxysilane-co-N-hydroxysuccinimide acrylate having a molecular weight of 250,000 was added, and the water bath was heated to 60 degrees Celsius for reaction for 12 hours. Then, washing was performed 3 times with anhydrous tetrahydrofuran.

The remaining steps were the same as those in Example 8.

Effect test: After the reaction was completed, the agarose beads were packed into a column, the protein loading capacity of the glass beads was tested using a histidine-tagged protein, and the protein loading capacity of the batch of glass beads was measured to be 2.2 mg/mL.

### Example 10

(1) Several glass beads having a diameter of 100 µm were weighed and placed into a 2,000 mL beaker, after ultrasonic soaking with 1 M sodium hydroxide, the beaker was maintained in a 70-degree Celsius water bath for 90 min, the glass beads were ultrasonically washed three times with deionized water until neutral (pH test paper), and finally subjected to displacement washing once with anhydrous ethanol.
(2) 1 L of the washed glass beads was taken; after ethanol displacement was performed (3 times), the obtained glass beads were transferred to a four-necked reaction vessel; after the paste-like glass beads were washed with ethanol and transferred, the ethanol was controlled at 5 L; the stirrer was turned on, and the stirring speed was adjusted to about 180 rpm; the temperature was raised to 60°C, 0.5 L of 3-aminopropyltriethoxysilane was added, the reaction was started, and the reaction was continued for 48 h; after completion of the reaction, washing was performed alternately with three times the volume of ethanol and water until the pH was neutral.
(3) 10 mL of the glass beads modified with 3-aminopropyltriethoxysilane were re-dispersed in 60 mL of 2-morpholinoethanesulfonic acid solution (pH = 6.0), and 0.39 g of sodium polyglutamate having a molecular weight of 700,000, 2.35 g of 1-ethyl-3-(3-dimethylaminopropyl)carbodiimide hydrochloride, and 1.64 g of N-hydroxysuccinimide were added, and the reaction was carried out at 37°C for 2 h. After completion of the reaction, washing was performed 4 times with 2-morpholinoethanesulfonic acid solution.
(4) 5 mL of the washed glass beads were re-dispersed in 30 mL of 2-morpholinoethanesulfonic acid solution (pH = 6.0), and 25 µL of propylenediamine, 0.23 g of 1-ethyl-3-(3-dimethylaminopropyl)carbodiimide hydrochloride, and 0.16 g of N-hydroxysuccinimide were added, and the reaction was carried out at 30°C for 2 h. After completion of the reaction, washing was performed 3 times with 2-morpholinoethanesulfonic acid solution.
(5) Then, 5 mL of the washed glass beads were re-dispersed in 20 mL of 2-morpholinoethanesulfonic acid solution (pH = 6.0), and 0.19 g of sodium polyglutamate having a molecular weight of 700,000, 2.35 g of 1-ethyl-3-(3-dimethylaminopropyl)carbodiimide hydrochloride, and 1.64 g of N-hydroxysuccinimide were added, and the reaction was carried out at 29°C for 2 h. After completion of the reaction, washing was performed 3 times with 2-morpholinoethanesulfonic acid solution.
(6) 5 mL of the washed glass beads were re-dispersed in 30 mL of 2-morpholinoethanesulfonic acid solution (pH = 6.0), and 30 µL of propylenediamine, 0.23 g of 1-ethyl-3-(3-dimethylaminopropyl)carbodiimide hydrochloride, and 0.16 g of N-hydroxysuccinimide were added, and the reaction was carried out at 30°C for 2 h. After completion of the reaction, washing was performed 3 times with 2-morpholinoethanesulfonic acid solution.
(7) After completion of the reaction, the obtained glass beads were washed 3 times with 2-morpholinoethanesulfonic acid solution, re-dispersed in 30 mL of 2-morpholinoethanesulfonic acid solution (pH = 5.0), and 0.15 g of biotin, 0.30 g of 1-ethyl-3-(3-dimethylaminopropyl)carbodiimide hydrochloride, and 0.21 g of N-hydroxysuccinimide were added, and the reaction was carried out at 25°C for 12 h.

Effect test: After the reaction was completed, the glass beads were packed into a column, the protein loading capacity of the glass beads was tested using a streptavidin-tagged protein, and the protein loading capacity of the batch of glass beads was measured to be 1.2 mg/mL.

### Example 11

(1) Several glass beads having a diameter of 40 µm were weighed and placed into a 2,000 mL beaker, after ultrasonic soaking with 1 M sodium hydroxide, the beaker was maintained in a 70-degree Celsius water bath for 90 min, the glass beads were ultrasonically washed three times with deionized water until neutral (pH test paper), and finally subjected to displacement washing once with anhydrous ethanol.
(2) 1 L of the washed glass beads was taken; after ethanol displacement was performed (3 times), the obtained glass beads were transferred to a four-necked reaction vessel; after the paste-like glass beads were washed with ethanol and transferred, the ethanol was controlled at 5 L; the stirrer was turned on, and the stirring speed was adjusted to about 180 rpm; the temperature was raised to 60°C, 0.5 L of 3-aminopropyltriethoxysilane was added, the reaction was started, and the reaction was continued for 48 h; after completion of the reaction, washing was performed alternately with three times the volume of ethanol and water until the pH was neutral.
(3) 10 mL of the glass beads modified with 3-aminopropyltriethoxysilane were re-dispersed in 60 mL of 2-morpholinoethanesulfonic acid solution (pH = 6.0), and 0.39 g of sodium polyglutamate having a molecular weight of 700,000, 2.35 g of 1-ethyl-3-(3-dimethylaminopropyl)carbodiimide hydrochloride, and 1.64 g of N-hydroxysuccinimide were added, and the reaction was carried out at 37°C for 2 h. After completion of the reaction, washing was performed 4 times with 2-morpholinoethanesulfonic acid solution.
(4) 5 mL of the washed glass beads were re-dispersed in 30 mL of 2-morpholinoethanesulfonic acid solution (pH = 5.0), and 28 µL of propylenediamine, 0.23 g of 1-ethyl-3-(3-dimethylaminopropyl)carbodiimide hydrochloride, and 0.16 g of N-hydroxysuccinimide were added, and the reaction was carried out at 30°C for 2 h.
(5) After completion of the reaction, the obtained glass beads were washed 3 times with 2-morpholinoethanesulfonic acid solution, re-dispersed in 30 mL of 2-morpholinoethanesulfonic acid solution (pH = 5.0), and 0.15 g of biotin, 0.30 g of 1-ethyl-3-(3-dimethylaminopropyl)carbodiimide hydrochloride, and 0.21 g of N-hydroxysuccinimide were added, and the reaction was carried out at 25°C for 12 h.

Effect test: After the reaction was completed, the glass beads were packed into a column, the protein loading capacity of the glass beads was tested using a streptavidin-tagged protein, and the protein loading capacity of the batch of glass beads was measured to be 1.1 mg/mL.

### Example 12

(1) Several glass beads having a diameter of 40 µm were weighed and placed into a 2,000 mL beaker, after ultrasonic soaking with 1 M sodium hydroxide, the beaker was maintained in a 70-degree Celsius water bath for 90 min, the glass beads were ultrasonically washed three times with deionized water until neutral (pH test paper), and finally subjected to displacement washing once with anhydrous ethanol.
(2) 1 L of the washed glass beads was taken; after ethanol displacement was performed (3 times), the obtained glass beads were transferred to a four-necked reaction vessel; after the paste-like glass beads were washed with ethanol and transferred, the ethanol was controlled at 5 L; the stirrer was turned on, and the stirring speed was adjusted to about 180 rpm; the temperature was raised to 60°C, 0.5 L of 3-aminopropyltriethoxysilane was added, the reaction was started, and the reaction was continued for 48 h; after completion of the reaction, washing was performed alternately with three times the volume of ethanol and water until the pH was neutral.
(3) 10 mL of the glass beads modified with 3-aminopropyltriethoxysilane were re-dispersed in 20 mL of 2-morpholinoethanesulfonic acid solution (pH = 3.5), and 2.36 g of polyacrylic acid having a molecular weight of 4,500,000, 2.35 g of 1-ethyl-3-(3-dimethylaminopropyl)carbodiimide hydrochloride, and 1.64 g of N-hydroxysuccinimide were added, and the reaction was carried out at 30 °C for 2 h. After completion of the reaction, washing was performed 4 times with 2-morpholinoethanesulfonic acid solution.
(4) 5 mL of the washed glass beads were re-dispersed in 30 mL of 2-morpholinoethanesulfonic acid solution (pH = 5.0), and 5.32 g of nitrilotriacetic acid disodium salt, 1.31 g of 1-ethyl-3-(3-dimethylaminopropyl)carbodiimide hydrochloride, and 0.91 g of N-hydroxysuccinimide were added, and the reaction was carried out at 25 °C for 12 h.
(5) After completion of the reaction, washing was repeated 3 times with 2-morpholinoethanesulfonic acid solution, the washed glass beads were immersed in 25 mL of 100 mM nickel sulfate solution, and the reaction was carried out with stirring at room temperature for 1 hour.

Effect test: After the reaction was completed, the glass beads were packed into a column, the protein loading capacity of the glass beads was tested using a histidine-tagged protein, and the protein loading capacity of the batch of glass beads was measured to be 0.12 mg/mL.

### Example 13: Large-scale preparation of biomicrospheres

(1) 5.1 kilograms of glass beads having a diameter of 40 µm were weighed and placed into a 6000 mL beaker, after ultrasonic soaking with 1 M sodium hydroxide, the beaker was maintained in a 70-degree Celsius water bath for 90 min, the glass beads were ultrasonically washed three times with deionized water until neutral (pH test paper), and finally subjected to displacement washing once with anhydrous ethanol.
(2) The washed glass beads were taken; after ethanol displacement was performed (3 times), the obtained glass beads were transferred to a four-necked reaction vessel; after the paste-like glass beads were washed with ethanol and transferred, the ethanol was controlled at 5.6 L; the stirrer was turned on, and the stirring speed was adjusted to about 200 rpm; the temperature was raised to 60°C, 1.2 L of 3-aminopropyltriethoxysilane was added, the reaction was started, and the reaction was continued for 48 h; after completion of the reaction, washing was performed alternately with three times the volume of ethanol and water until the pH was neutral.
(3) 5 kilograms of the glass beads modified with 3-aminopropyltriethoxysilane were re-dispersed in 10 L of 2-morpholinoethanesulfonic acid solution (pH = 4.1), and 90 g of sodium polyacrylate having a molecular weight of 150,000, 175 g of 1-ethyl-3-(3-dimethylaminopropyl)carbodiimide hydrochloride, and 265 g of N-hydroxysuccinimide were added, and the reaction was carried out at 30°C for 2 h. After completion of the reaction, washing was performed 4 times with 2-morpholinoethanesulfonic acid solution.
(4) The washed glass beads were re-dispersed in 9.5 L of 2-morpholinoethanesulfonic acid solution (pH = 5.0), and 1,172 g of nitrilotriacetic acid disodium salt, 287 g of 1-ethyl-3-(3-dimethylaminopropyl)carbodiimide hydrochloride, and 401 g of N-hydroxysuccinimide were added, and the reaction was carried out at 25°C for 12 h.
(5) After completion of the reaction, washing was repeated 3 times with 2-morpholinoethanesulfonic acid solution, the washed glass beads were immersed in 25 mL of 100 mM nickel sulfate solution, and the reaction was carried out with stirring at room temperature for 1 hour.

Effect test: After the reaction was completed, the glass beads were packed into a column, the protein loading capacity of the glass beads was tested using a histidine-tagged protein, and the protein loading capacity of the batch of glass beads was measured to be 3.9 mg/mL.

It may be seen from the above examples that the preparation process of the biomicrosphere of the present disclosure is simple and the conditions are mild, the obtained biomicrosphere has good adsorption performance, and is suitable for large-scale production.

The above are only some examples of the present disclosure, and the present disclosure is not limited solely to the contents of the above examples.

All documents mentioned in the present disclosure are incorporated in the present application by reference as if each document is cited separately as a reference. In addition, it should be understood that, after reading the above contents of the present disclosure, those skilled in the art may make various changes or modifications to the present disclosure, and these equivalent forms likewise fall within the scope defined by the appended claims of the present application.

## Claims

1. A biomicrosphere, wherein the outer surface of a microsphere body of the biomicrosphere is covalently coupled with at least one polymer chain, and other portions of the polymer chain are unattached to the outer surface of the microsphere body; the polymer chain contains a functional group, and the functional group specifically binds to a biological target via a specific binding site, wherein the polymer chain comprises at least one segment, each of the at least one segment is derived from a polymer, and the at least one segment of the polymer chain is derived from a polymer having a molecular weight > 5000; the particle size of the biomicrosphere body is ≥ 10 µm.

2. The biomicrosphere according to claim 1, wherein the polymer is a linear polymer, preferably, the linear polymer has a branched chain, and the end of the branched chain contains the functional group; and/or
the functional group of the polymer is one of carboxyl, hydroxyl, amino, and sulfhydryl or a combination thereof.

3. The biomicrosphere according to claim 1 or 2, wherein a monomer of the polymer is an acrylic polymer, an acrylic copolymer, an amino acid polymer, or other carboxylic acid polymers; preferably, a monomer unit of the acrylic polymer comprises one of acrylic acid, acrylate salt, acrylate ester, methacrylic acid, methacrylate salt, and methacrylate ester or a derivative thereof, or any combination of the above.

4. The biomicrosphere according to claim 1, wherein the functional group is linked to the specific binding site;
preferably, the specific binding site comprises a nickel ion, biotin, a biotin analog, avidin, an avidin analog, an antibody-type tag, or an antigenic tag.

5. The biomicrosphere according to any one of claims 1-4, wherein the molecular weight of the polymer is: greater than or equal to 50,000, greater than or equal to 150,000, 450,000-6,000,000, or 450,000-4,500,000, preferably, the molecular weight of the linear polymer is 50,000, 150,000, 200,000, 300,000, 400,000, 500,000, 600,000, 700,000, 800,000, 900,000, 1,000,000, 1,250,000, 1,500,000, 1,800,000, 2,000,000, 2,500,000, 3,000,000, 3,500,000, 4,000,000, 4,500,000, 5,000,000, or 6,000,000; and/or
the particle size of the biomicrosphere body is 10 µm-600 µm, 30 µm-600 µm, greater than or equal to 30 µm and less than 600 µm; preferably, the particle size of the biomicrosphere body is 10 µm, 40 µm, 50 µm, 60 µm, 70 µm, 80 µm, 90 µm, 100 µm, 120 µm, 150 µm, 200 µm, 300 µm, 400 µm, and 600 µm.

6. The biomicrosphere according to any one of claims 1-5, wherein the biomicrosphere is free of network cross-linking.

7. The biomicrosphere according to any one of claims 1-6, wherein the coupling of the polymer chain to the surface of the microsphere body is achieved by covalently coupling the polymer which is known to the outer surface of the microsphere body;
for a polymer chain having a plurality of the segments, one known polymer is covalently coupled to the outer surface of the microsphere body and then linked to another known polymer.

8. The biomicrosphere according to any one of claims 1-7, further having any one or more of the following features:
(1) when the polymer chain comprises a plurality of segments, the polymer from which each segment is derived is the same or different;
(2) the microsphere body is a solid microsphere;
(3) the microsphere body is a glass microsphere or a microsphere containing a magnetic material.

9. A method for preparing a biomicrosphere, wherein
a known polymer is covalently coupled to a surface of a microsphere body to achieve covalent coupling between the surface of the microsphere body and a polymer chain;
preferably, no crosslinking agent is used and/or the polymer is a linear polymer.

10. The method for preparing the biomicrosphere according to claim 9, comprising the following steps:
(1) subjecting the biomicrosphere body to chemical modification, introducing amino or other groups capable of binding to carboxyl onto the outer surface of the biomicrosphere body, to form a biomicrosphere A;
preferably, the biomicrosphere body is chemically modified using a silane coupling agent;
(2) under the condition of no crosslinking agent, linking a polymer to a surface of the biomicrosphere A to obtain a biomicrosphere B.

11. The method for preparing the biomicrosphere according to claim 9, comprising the following steps:
linking the silane coupling agent to the polymer, and then linking the resulting product to the biomicrosphere body to obtain the biomicrosphere B.

12. The method for preparing the biomicrosphere according to claim 10 or 11, further comprising:
(3) under the condition of no crosslinking agent, further linking other polymers to the surface of the biomicrosphere B to obtain a biomicrosphere D.

13. The method for preparing the biomicrosphere according to any one of claims 9-12 in large scale, further comprising the following steps: carrying out a reaction of linking a functional group of the polymer to a specific binding site, preferably, using the biomicrosphere B or the biomicrosphere D to carry out the reaction of linking the functional group of the polymer to the specific binding site.

14. The method for preparing the biomicrosphere according to claim 13 in large scale, wherein the biomicrosphere B or the biomicrosphere D is coupled with tricarboxyamine, and then complexed with Ni ions to obtain a biomicrosphere C;
or, biotin, a biotin analog, avidin, an avidin analog, an antibody-type tag, or an antigenic tag is linked to the functional group;
preferably, the tricarboxyamine is N,N-bis(carboxymethyl)-L-lysine, nitrilotriacetic acid, and a combination thereof.

15. The method for preparing the biomicrosphere according to any one of claims 9-14 in large scale, wherein the ratio of the usage amount of the linear polymer to the biomicrosphere body is: 8 × 10⁻⁷ mol/10 mL-2.0 × 10⁻⁵ mol/10 mL.

16. A method for separating a biological target, wherein the biomicrosphere according to any one of claims 1-8 is used to bind to the biological target to be separated.

17. The method for separating the biological target according to claim 16, wherein the biological target is one or more of a protein, a nucleic acid, a cell, a polysaccharide, and a peptide.

18. A separation system, wherein the separation system uses the biomicrosphere according to any one of claims 1-8, or uses the biomicrosphere prepared by the method according to any one of claims 9-15 for separation.
